Europäisches Patentamt

(19) European Patent Office

Office européen des brevets

(11) Publication number : **0 243 321 B1**

(12) EUROPEAN PATENT SPECIFICATION

(45) Date of publication of patent specification :
02.10.91 Bulletin 91/40

(51) Int. Cl.⁵ : **A61J 1/00, A61J 7/00, B65D 35/00**

(21) Application number : 87830030.0

(22) Date of filing : 26.01.87

(54) **Packing system for a habituation-free use of products for topical application such as emulsions, creams, unguents, pastes, salves, gels, lipogels and the like.**

(30) Priority : 27.01.86 IT 511586

(43) Date of publication of application :
28.10.87 Bulletin 87/44

(45) Publication of the grant of the patent :
02.10.91 Bulletin 91/40

(84) Designated Contracting States :
AT BE CH DE ES FR GB GR LI LU NL SE

(56) References cited :
DE-A- 2 002 622
DE-C- 624 523
FR-A- 781 074
GB-A- 642 692
GB-A- 1 195 534

(73) Proprietor : KEMINOVA ITALIANA S.R.L.
Via G. Garibaldi, 19
I-25082 Botticino Sera (Brescia) (IT)

(72) Inventor : Mora,Valtiero Dr.
Via Zamboni,97
25100 Brescia (IT)

(74) Representative : Massari, Marcello
Studio M. Massari S.r.l. 23, Via Fontanella
Borghese
I-00186 Roma (IT)

## Description

This invention relates to a packing system of products for topical application such as emulsions, creams, unguents, pastes, salves, gels, lipogels and the like, wherein two or more products are arranged in distinct layers, within a single container. An example of such a packing system is disclosed in GB-A-642 692. The aim of the therein described stratified arrangement of two different product is however only that of avoiding the wastage of expensive products which are packed in collapsible tubular containers.

According to the invention, in stead, the products arranged in distinct layers in a single container, are distributed and applied consecutively in order to alternate the use thereof or to utilize them in progressively increasing concentrations to avoid habituation in periodic or cyclic applications.

In the field of dermatology, cosmetic and medical surgical aids it is common to use products for topical applications such as emulsions, creams, unguents, pastes, salves, gels, lipogels and the like. Unfortunately these products for topical use, containing active components often give rise to a worrying, at sight not explicable phenomenon.

This is the case, for example, for dermatological and cosmetic products. In fact it happens that many consumers continually using functional dermocosmetic products containing active cosmetic substances such as to promote eudermic effects, like for instance hydrating, nourishing, bioregenerating products for hardening and rejuvenating the skin and products for the body, notice after an appreciable initial improvement that the signs of further getting better cease completely, although they constantly and protractdely continue using the same products. This results more evident and stronger especially when effective and complete products are used.

This phenomenon well-known in the pharmacological, biochemical field should be explained by the habituation or addiction to substances, i.e. a particular form of adaption of the living organism which is common to all human being and is established more or less slowly as a defence of the organism itself against exterior agents in case of repeated and constant giving of heterogeneous substances as well as biologically and pharmacologically active principles, whereby the living organism becomes always less and less sensitive and reactive to such substances and requires even greater and progressively increasing doses of such active principles to achieve and keep steady the same effects and responses.

The functional cosmetic products can be grouped in two classes:

a) products of continuous and daily use, like for example day and night creams; and
b) superactive shock products of periodic or cyclic use.

Products of continuous use, which are daily applicated too long and are composed of substances having essentially always the same concentration, cause in the cells of the skin a phenomenon socalled "biological nausea".

In fact, active principles both of synthetic and animal origin used too long cause in the cells a state of dull reactivity with not yet well-known processes leading to a partial block of bioenzymatic and osmotic systems governing the adsorption of such active principles with the consequence of a changed local chemism and an impossible integration of such active principles in the metabolism of the skin.

In such a case, the continuous and protracted use causes the phenomenon of the habituation or addiction to such active principles with the consequence of a decrease first, then a disappearance of the effectiveness of the used product.

The sole, logic, obvious remedy for this defect is alternately using different products which are composed of completely different substances, if possible, belonging to different kingdoms, i.e. vegetable and animal, although they aim at the same cosmetic purpose (for exaple, protective, hydrating, nourishing, regenerating purpose).

With such an extremely rational system, skin is allowed to keep an always live and quick reactivity without the danger of its weakening or extinguishing as it is excited by the alternance of different active products which stimulate it in a different way.

In case of superactive or shock products to be periodically applied, use of active principles, applied in great and always the same doses with the aim at achieving quicker results, causes in the cells after a short initial getting better an indesired phenomenon socalled "biological exhaustion", characterised in that cells cannot keep a long time high levels of unnatural reactivity, whereby after a more or less short period of time the desired eudermic effect does not take place any longer.

The cause of this great defect is the exaustion, block or modification of particular cellular receptors normally exciting the reaction to bioactive stimuli. In such cases skin should come in contact with greater and greater doses of active principles in order to react again, the peculiar characteristic of the habituation being the establishing of the resistance to the dose and not to the active principle.

Consequently, it is necessary to give greater and greater doses to the organism to achieve the same effect or to stop the treatment for some day to allow the organism to become sensitive to the action of such substances again.

In fact, the habituation usually is only transitory. In this case, when using superactive impact cosmetics to be daily but periodically applied, the most logic remedy to avoid the danger of habituation would be that of applying in each (regenerating, anti-wrinkling, revitalizing, hardening) treatment small and different quantities of product having increasing concentration of the same active principle so as to increasingly grade the doses for the whole treatment. Starting from such statement the object of this invention is to provide a packing system and a method of using products for topical application in order to advantageously allow the serious phenomenon of the habituation or habit leading to the inefficacy of the used product to be avoided.

Another object of the invention is to place at users disposal confections containing various products proportioned so as to automatically achieve a dermatologic or cosmetic treatment without causing habituation but by further granting the efficiency of the active principles of the products.

To this end, according to the present invention, a packing system is provided which is characterized by the features defined in claim 1.

Further details of this invention will become apparent from the following description with reference to the accompanying indicative and not limitative drawing, in which:

Fig. 1 shows a section of a container made up with two products;

Fig. 2 is a similar view of a container made up with a plurality of layers of different products.

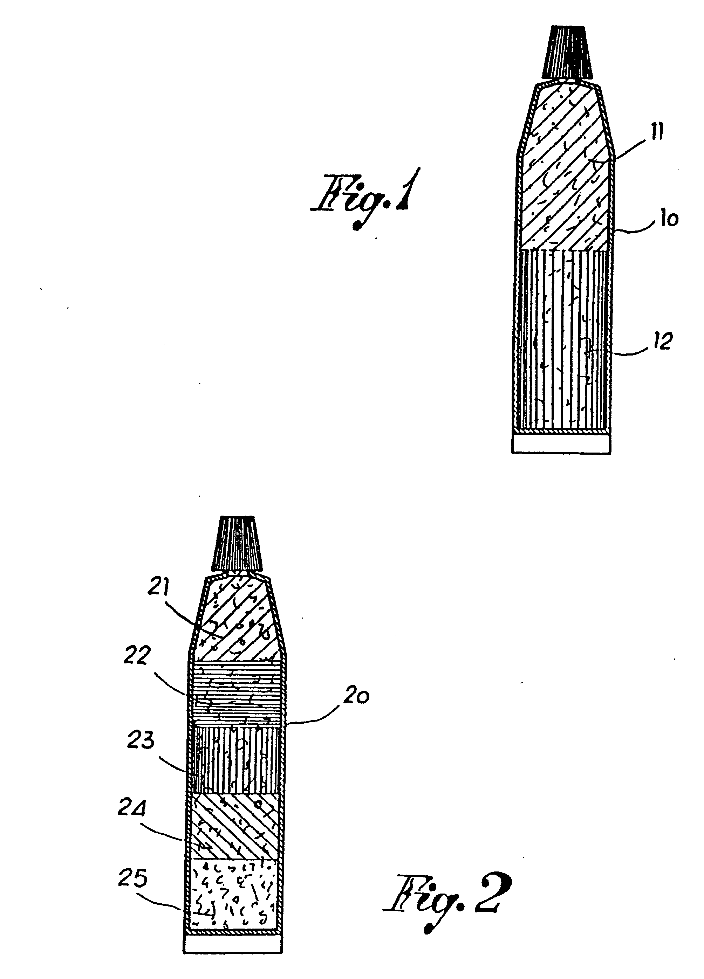

Dermatologic and cosmetic products of daily and continuous use, like for example day and night creams, eye-cream, regenerating creams for sensitive and couperose-affected skin, hardening, shrinking, anti-cellulitis products for the body, tooth pastes for the oral hygiene and the care of the teeth and gums, are contained in confections 10 (Fig. 1) containing at least two separate products 11-12 in the same or different quantity but being different as active substancea aiming at the same dermatologic or cosmetic purpose.

The two or more products 11-12 contained in the same container can be different both as for active substances and type, appearance, smell, colour and, in case of products for the oral hygiene, taste and flavour, in order to make a visual, olfactory and gustatory distinction.

Considering, for example, a cosmetic product which is continually used every day such as a revitalizing night-cream, two creams seoarate from each other, for example the first white, the second pink, are filled in the same container such as a metallic or plastic tube essentially in the same quantity, each of them however, being composed of qualitatively different revitalizing substances.

In this way two different creams are delivered one after the other, for example, the white cream first, and then the pink cream when the white is over. When the pink cream also is over, a new tube of regenerating night-cream will be applied so as to still deliver the white cream first, and then the pink cream a.s.o.

In this way an alternate use of different products is granted so that the skin is not allowed to accustom itself to the different active substances contained in both creams, thus avoiding the habituation.

A similar packing system can be also provided for dermatologic and cosmetic products of periodic use, for example , for a treatment of two to eight applications in a period of one year with revitalizing, regenerating, anti-wrinkling, hardening, shrinking, impact or shocking substances, and anti-vibex, anti-cellulitis products as well-as special tooth pastes for the care of teeth and gums.

As above mentioned, organism accustoms itself to the concentration or dose of the active principle so that greater and greater doses are needed to achieve and keep the same initial effect. Furthermore the habituation is transitory.

Thus in case of a treatment of progressively increasing intensity more equal or different lavers 21, 22, 23, 24, 25 of a product having an increasing concentration of active principles from the first to the last one are filled in the same container 20 (Fig. 2). Therefore a product in stronger and stronger doses or concentrations is delivered-from only one container to avoid the habituation of the body. In the course of the treatment a change in the colour intensity of the product, for example from white to deep pink, indicates visually the progressive increase of the doses of active substances.

## Claims

1. Packing system of products for topical application such as emulsions, creams, unguents, pastes, salves, gels, lipogels and the like, which provides the arrangment in distinct layers, within a single container(s), of two or more products (11, 12) either in different or equal amount, whereby the products in layers are delivered and used consecutively, characterized in that said two or more products are differentiated one from the other by either their compositions and/or the different type and/or different concentrations of active substances which they contain, in order to alternate the application or to apply progressively increasing doses and concentrations of said active substances, thus ensuring an habituation-free use of said active substances.

2. The system of claim 1, wherein the two or more products (11-12) arranged in the same container and delivered one after the other are composed of different substances preferably of different either vegetable or animal origin.

3. The system of claim 2, wherein said products are coloured in a different way for the visual identification of the kind of product as far as the appearance the smell, the taste and the flavour is concerned.

4. The system of claim 1, wherein the two or more products (21, 22, 23, 24,25) arranged in the same container and delivered one after the other contains doses of active substances having concentrations increasing from the first to the last layers.

5. The system of claim 4, wherein said products are coloured in a different way for the visual identification and are prepared with either equal or different substances.

## Patentansprüche

1. Verpackungssystem von Produkten zur topischen Anwendung wie etwa Emulsionen, Cremes, Unguenta, Pasten, Salben, Gele, Lipogele und dergleichen, bei dem in einem einzigen Behälter (10) zwei oder mehr Produkte (11, 12) entweder in verschiedenen oder gleichen Mengen in voneinander getrennten Lagen angeordnet sind, so daß die in Lagen angeordneten Produkte nacheinander abgegeben und verwendet werden, dadurch gekennzeichnet, daß sich die zwei oder mehr Produkte entweder durch ihre Zusammensetzungen und/oder die verschiedene Art und/oder verschiedene Konzentrationen von darin enthaltenen wirkstoffen unterscheiden, um die Anwendung zu wechseln oder zunehmend größere Dosen und Konzentrationen des wirkstoffs anzuwenden, wodurch eine gewöhnungsfreie Anwendung des wirkstoffs gewährleistet ist.

2. System nach Anspruch 1, wobei die zwei oder mehre Produkte (11-12), die im gleichen Behälter angeordnet sind und nacheinander abgegeben werden, aus verschiedenen Stoffen zusammengesetzt sind, die bevorzugt verschiedenen pflanzlichen oder tierischen Ursprungs sind.

3. System nach Anspruch 2, wobei die Produkte zur visuellen Erkennung der Art des Produkts hinsichtlich des Aussehens, des Geruchs, des Geschmacks und des Aromas verschiedenfarbig sind.

4. System nach Anspruch 1, wobei die zwei oder mehr im gleichen Behälter angeordneten und nacheinander abgegebenen Produkte (21, 22, 23, 24, 25) wirkstoffdosen enthalten, deren Konzentrationen von der ersten zur letzten Lage zunehmen.

5. System nach Anspruch 4, wobei die Produkte zur visuellen Erkennung verschiedenfarbig und entweder mit gleichen oder verschiedenen Stoffen hergestellt sind.

## Revendications

1. Dispositif d'emballage de produits pour l'application topique de par exemple des émulsions, des crèmes, des onguents, des pâtes, des pommades, des gels, des lipogels et analogues, qui permet d'obtenir la disposition en couches distinctes, à l'intérieur d'un même conteneur (10), de deux ou plusieurs produits (11, 12) en quantités soit différentes, soit égales, de façon que les produits en couches soient appliqués et utilisés consécutivement, dispositif caractérisé en ce que les deux produits ou plus sont différenciés les uns des autres par leurs compositions et/ou leurs types différents et/ou les concentrations différentes des substances actives qu'ils contiennent, de manière à alterner l'application ou à appliquer progressivement des doses et des concentrations croissantes de ces substances actives pour assurer ainsi un usage sans accoutumance de ces substances actives.

2. Dispositif selon la revendication 1, caractérisé en ce que les deux produits ou plus (11-12) placés dans le même conteneur et fournis l'un après l'autre, sont composés de substances différentes et de préférence d'origines différentes soit végétales, soit animales.

3. Dispositif selon la revendication 2, caractérisé en ce que les produits sont colorés de manières différentes pour permettre l'identification visuelle du type de produit en ce qui concerne l'aspect, l'odeur, le goût et le parfum.

4. Dispositif selon la revendication 1, caractérisé en ce que les deux produits ou plus (21, 22, 23, 24, 25) placés dans le même conteneur et fournis les uns après les autres, contiennent des doses de substances actives présentant des concentrations croissantes de la première à la dernière couches.

5. Dispositif selon la revendication 4, caractérisé en ce que les produits sont colorés de manières différentes pour permettre l'identification visuelle, et préparés avec des substances soit égales, soit différentes.

*Fig.1*

*Fig.2*